# EUROPEAN PATENT APPLICATION

(11) **EP 1 454 578 A2**
(43) Date of publication of application: **08.09.2004**
(21) Application number: 04250626.1
(22) Date of filing: 05.02.2004
(51) Int. Cl.: A47K 10/38

(54) **Wet sheet container**

(30) Priority: 07.03.2003 JP 2003060854
(71) Applicant: Uni-Charm Corporation, Shikokuchuo-shi, Ehime-ken (JP)
(72) Inventor: Saitou, Ikuya, Kawanoe-shi Ehime-ken (JP); Miyazawa, Kiyoshi, Kawanoe-shi Ehime-ken (JP)
(74) Representative: Fitchett, Stuart Paul

(57) **Abstract**

Disclosed is a wet sheet container for housing a sheet roll that is a wet sheet wound in the form of a roll. A container body is formed with a storage space into which the sheet roll is to be inserted. The container body is separable into two or more parts so that separation of the container body allows insertion of the sheet roll into the storage space. A shaft is located inside the container body so as to be inserted into a center hole of the sheet roll. The shaft defines a support space which extends along an axial direction thereof and communicates with the outside of the container body.

## Description

### BACKGROUND OF THE INVENTION

### Field of the Invention

The present invention relates to a wet sheet container for housing a wet sheet that is wound in the form of a roll, more particularly, relates to a wet sheet container that can be attached to a toilet paper holder.

### Description of the Related Art

Wet sheets impregnated with chemicals such as sterilizer, detergent and humectant have been widely used in homes. Particularly when set in bathrooms, they can be conveniently used for wiping buttocks or for cleaning toilets. However, unlike ordinary toilet paper and dry tissue paper, they need be hermetically housed in a container, except in use, so as not to be exposed to the outside air for preventing the chemicals from drying. Heretofore, such wet sheets have been hermetically housed in a container that is in the form of a hexahedral box or cylinder. Such a hexahedral or cylindrical container is designed to be placed on a floor or shelf, but it is structurally difficult to attach the container to a toilet paper holder or put the container on a wall surface other than the bathroom.

Japanese Unexamined Patent Publication No. 2001-95718 (Patent Publication 1) discloses a wet tissue container which is to be attached to a cutter of a toilet paper holder and a cylindrical container for a roll of wet tissue which is to be attached to a toilet paper holder instead of a toilet paper such that the holder is inserted into a center hole of the container.

Japanese Unexamined Patent Publication No. H09-149863 (Patent Publication 2) discloses an airtight container for a roll of wet tissue which is to be fitted in a base secured to a mounting surface of a wall by means of screws or the like.

However, the first container of the Patent Publication 1, which is to be attached to a cutter of a toilet paper holder, is unstable in position, wherein the container may be turned upwardly along with the cutter due to a force for pulling a tissue paper out of the container, so that the tissue paper cannot be pulled out smoothly. In addition, since it is troublesome to insert the curved cutter into the container, the attachment and detachment of the container will be difficult to perform.

The second container of the Patent Publication 1, which is cylindrical, can be readily attached to a toilet paper holder. However, the cylindrical container may freely rotate on the toilet paper holder, so that the tissue paper cannot be pulled out smoothly. In addition, if the center hole is simply formed in the container, the center hole will impair airtightness of the container, resulting in drying of the tissue paper inside thereof.

In the invention disclosed in the Patent Publication 2, on the other hand, it is troublesome to secure the dedicated base to a mounting surface of a wall by means of screws or the like. Furthermore, since the screws for securing the base need screw holes in the wall, the wall and wall paper will be damaged seriously.

### SUMMARY OF THE INVENTION

The present invention has been worked out in view of the shortcomings in the prior art set forth above. It is therefore an object of the present invention to provide a wet sheet container which can be readily set without a dedicated mounting base as well as can keep its airtightness.

According to the present invention, there is provided a wet sheet container for housing a sheet roll that is a wet sheet wound in the form of a roll, comprising:
a container body formed with a storage space into which the sheet roll is to be inserted, the container body being separable into two or more parts so that separation of the container body allows insertion of the sheet roll into the storage space; and
a shaft located inside the container body so as to be inserted into a center hole of the sheet roll, the shaft defining a support space which extends along an axial direction thereof and communicates with the outside of the container body.

The wet sheet container of the present invention can be readily set on a wall such as by inserting a rod of a toilet paper holder into the support space. Since the support space is separated from the storage space by the shaft, airtightness of the storage space can be kept even though the support space communicates with the outside of the container such as for insertion of the rod. Therefore, the wet sheet can be prevented from drying. In addition, since the sheet roll is rotatably attached to the shaft inside the storage space, the wet sheet can be easily pulled out.

In the wet sheet container of the present invention, for example, the container body may be separable in the axial direction of the shaft so that separation of the container body allows insertion of the sheet roll into the storage space along an axial direction of the sheet roll, and the container body formed with a dispensing opening for pulling out the wet sheet. In this construction, since the shaft can be inserted into the center hole of the sheet roll simultaneously with the insertion of the sheet roll into the storage space along the axial direction of the sheet roll, the sheet roll can be easily housed in the container.

In an alternative, the container body may be separable in a direction perpendicular to the axial direction of the shaft so that separation of the container body allows insertion of the sheet roll into the storage space together with the shaft inserted into the center hole of the sheet roll. In this construction, the insertion of the sheet roll can be performed by holding the shaft without touching the sheet roll. Here, when the wet tissue is to be used, the sheet roll may be exposed externally by separation of the container body so that the wet sheet can be directly unwound from the exposed sheet roll, or the container body may be formed with a dispensing opening for pulling out the wet sheet so as to avoid separation of the container body during use.

If a lid is provided to close the dispensing opening, the wet sheet stored in the storage space can be prevented from drying more effectively.

The wet sheet container of the present invention may be constructed such that the support space formed inside the shaft allows insertion of a rod of a toilet paper holder, and the container body has a rear side formed with an intermediate portion and support portions at opposite sides of the intermediate portion in a rotational direction about an axis of the shaft, the support portions being located further away from the axis of the shaft than is the intermediate portion, functioning to prevent rotation of the container body. In this construction, abutment of the support portions against the toilet paper holder or the like can prevent rotation of the container body, so that undesirable rotation of the container body during pulling out of the wet sheet can be prevented.

In the wet sheet container of the present invention, furthermore, a paper support member for supporting a core of a toilet paper may be provided outside the container body. In this construction, an ordinary toilet paper can also be provided even after the wet sheet container of the present invention is attached to a toilet paper holder.

### BRIEF DESCRIPTION OF THE DRAWINGS

The present invention will be understood more fully from the detailed description given hereinafter and from the accompanying drawings of the preferred embodiments of the present invention, which, however, should not be taken to be limitative to the invention, but are for explanation and understanding only.

In the drawings:
Fig. 1 is a perspective view showing a wet sheet container according to a first embodiment of the present invention, along with a sheet roll and a toilet paper holder;
Fig. 2 is a longitudinal sectional view showing a state where a container body shown in Fig. 1 is attached to the holder;
Fig. 3 is a transverse sectional view showing a state where the container body shown in Fig. 1 is attached to the holder;
Figs. 4A and 4B are perspective views showing a container body according to a second embodiment of the present invention;
Fig. 5 is a transverse sectional view showing a state where the container body shown in Fig. 4A or 4B is attached to the holder;
Fig. 6 is a longitudinal sectional view of a container body according to a third embodiment of the present invention;
Fig. 7 is a longitudinal sectional view of a container body according to a fourth embodiment of the present invention;
Fig. 8 is a perspective view showing a container body according to a fifth embodiment of the present invention; and
Fig. 9 is a perspective view showing a container body according to a sixth embodiment of the present invention.

### DESCRIPTION OF THE PREFERRED EMBODIMENT

The present invention will be discussed hereinafter in detail in terms of the preferred embodiments according to the present invention with reference to the accompanying drawings. In the following description, numerous specific details are set forth in order to provide a thorough understanding of the present invention. It will be obvious, however, to those skilled in the art that the present invention may be practiced without these specific details. In other instance, well-known structures are not shown in detail in order to avoid unnecessary obscurity of the present invention.

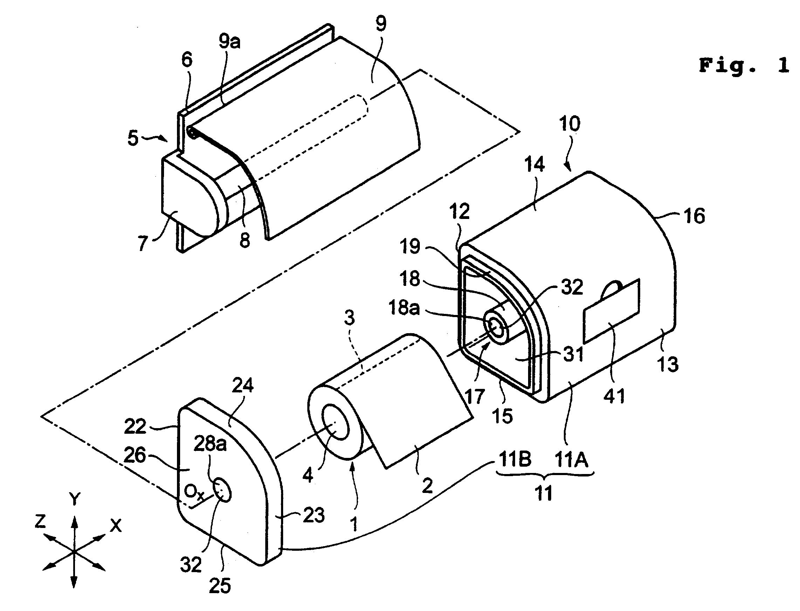

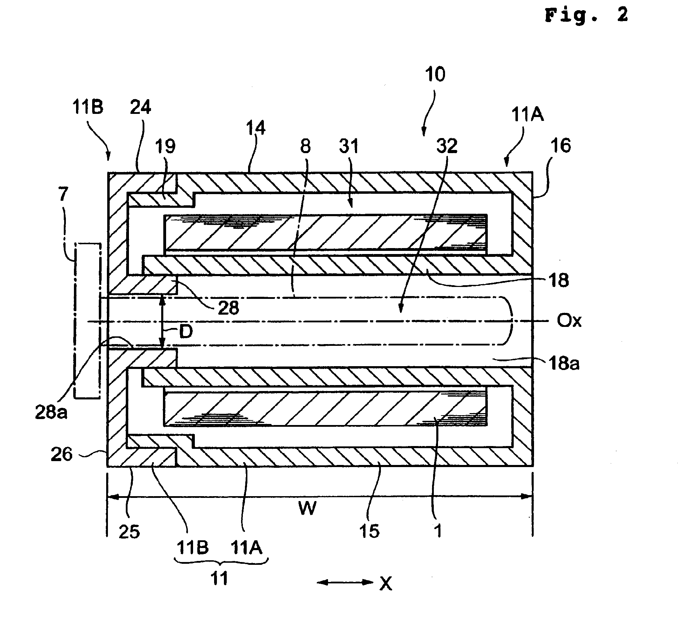

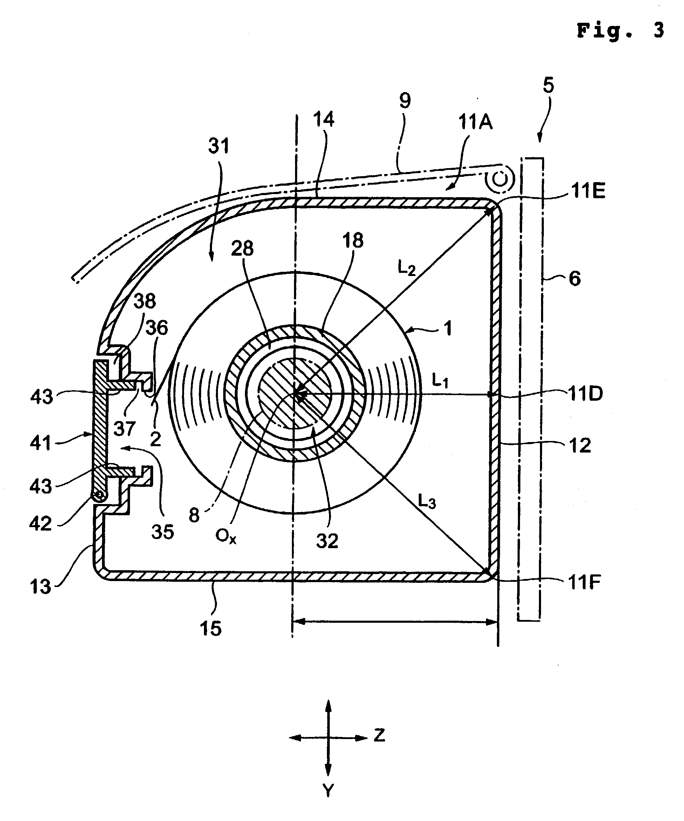

Fig. 1 is an exploded perspective view showing a wet sheet container 10 according to a first embodiment of the present invention, along with a sheet roll 1 which is to be housed in the wet sheet container 10 and a toilet paper holder5 to which the wet sheet container 10 is to be attached; Fig. 2 is a longitudinal sectional view taken along an XY-plane inclusive of an axis Ox-Ox, showing a state where the wet sheet container 10 is attached to the holder 5; and Fig. 3 is a transverse sectional view taken along a YZ-plane.

The wet sheet container 10 comprises a container body 11. The container body 10 is separable into a first casing 11A and a second casing 11B, wherein the separation direction is along the axis Ox-Ox.

The first casing 11A comprises a rear panel 12 to be opposed to the toilet paper holder 5, a front panel 13 for facing a user, a top panel 14, a bottom panel 15 and an end panel 16, which are formed integrally with each other, wherein an opening 17 is formed on the opposite side to the end panel 16. Inside the first casing 11A, as shown in Fig. 2, a shaft 18 is provided to extend along the axis Ox-Ox. The shaft 18 is formed integrally with the end panel 16. The shaft 18 is of a cylindrical configuration, defining therein a center hole 18a which extends along the axis OX-OX and opens on an outer surface of the end panel 16.

At the opening 17 of the first casing 11A, outer surfaces of the rear panel 12, the front panel 13, the top panel 14 and the bottom panel 15 are stepped toward the axis Ox-Ox to provide a fitting rib 19.

The second casing 11B is of a length much shorter than the length of the first casing 11A, as measured in a direction along the axis OX-OX. The second casing 11B comprises a rear panel 22, a front panel 23, a top panel 24, a bottom panel 25 and an end panel 26. Inside the second casing 11B, as shown in Fig. 2, a shaft 28 is integrally formed to project from the end panel 26 along the axis Ox-Ox, wherein a center hole 28a axially formed in the shaft 28 opens on an outer surface of the end panel 26.

The first casing 11A and the second casing 11B can engage one another as shown in Fig. 2. In this state, the shaft 28 of the second casing 11B is fitted into the center hole 18a of the shaft 18 of the first casing 11A, while the rear panel 22, the front panel 23, the top panel 24 and the bottom panel 25 of the second casing 11B make a fit with an outer surface of the fitting rib 19 of the first casing 11A.

In the container body 11 thus assembled, the panels 12, 13, 14, 15, 16, 22, 23, 24, 25, 26 define a storage space 31 around the shafts 18 and 28. With this fitting structure, the storage space 31 can be shut off from the outside air so as to be almost airtight. In this fitting structure, moreover, the center hole 18a of the shaft 18 of the first casing 11A and the center hole 28a of the shaft 28 of the second casing 11B are in communication with each other, defining a support space 32. The support space 32 opens on the respective outer surfaces of the end panels 16 and 26 to communicate with the outside of the container body 11. It should be noted that since the shafts 18 and 28 engage one another as shown in Fig. 2, the support space 32 is separated from the storage space 31 by the shafts 18 and 28, thereby keeping the airtightness of the storage space 31.

As shown in Figs. 1 and 3, a dispensing opening 35 is formed in the front panel 13 of the first casing 11A. Adjacent the dispensing opening 35, a restriction hole 36 opens into the storage space 31, as shown in Fig. 3. The restriction hole 36 is of an opening area that is smaller than (e.g., equal to or less than 1/2) the whole area of the container body 11 as viewed from its front side in the Z-direction. As the wet sheet is pulled out through the restriction hole 36, the restriction hole 36 provides a resistance to the wet sheet so that the wet sheet can be easily torn along perforation.

In front of the restriction hole 36, a shoulder 37 is formed, and in front of the shoulder 37, a recess 38 is formed to accommodate a lid 41. The lid 41 is pivoted at the lower end about a shaft 42 inside the recess 38. A fitting rib 43 is formed to project from an inner surface of the lid 41. When the lid 41 is closed as shown in Fig. 3, the fitting rib 43 is elastically deformed to fit into the shoulder 37, thereby preventing undesirable opening of the lid 41. With the lid 41 thus closing the dispensing opening 35, therefore, the airtightness of the storage space 31 can be certainly maintained.

As shown in Fig. 3, the rear panel 12 of the first casing 11A and the rear panel 22 of the second casing 11B provide flat surfaces parallel to the XY-plane. Consequently, the outer surfaces of the rear panels 12 and 22 provide an intermediate portion 11D, which is located a smallest distance L1 away from the axis Ox-Ox, and support portions 11E, 11F, which are located at opposite sides of the intermediate portion 11D in a rotational direction about the axis Ox-Ox and largest distances L2, L3 away from the axis Ox-Ox. It should be noted that the distances L2, L3, which may or may not be equal to each other, need be larger than the distance L1.

Since the support portions 11E, 11F are provided at opposite sides of the intermediate portion 11D, the container body 11 itself functions to prevent rotation.

The first casing 11A, the second casing 11B and the lid 41 forming the container body 11 may be formed by injection molding a synthetic resin material such as polypropylene (PP) resin, polyethylene (PE) resin, polystyrene (PS) resin, acrylonitrile-butadien-styrene (ABS) resin, elastomer resin, polyethylene terephthalate (PET) resin, polyvinyl chloride (PVC) resin, and polycarbonate resin.

The sheet roll 1 is a wet sheet 2 that is wound in the form of a roll to have a center hole 4 along its axis. Moreover, perforations 3 are formed in the wet sheet 2 at regular intervals in the longitudinal direction thereof so that it can be torn along the perforation 3 into small pieces as needed.

The wet sheet 2 may be a thin paper (tissue paper) manufactured by wet papermaking process with pulp as stock or a spunlaced nonwoven fabric manufactured by wet laying stock such as pulp and regenerated cellulose fibers and then subjecting it to a water jet treatment for fiber entanglement, wherein a water-soluble or water-swellable binder is preferably added to make the wet sheet 2 disintegratable in water. Alternatively, the wet sheet 2 may be a composite sheet of the thin paper or nonwoven fabric and a water-soluble resin film such as polyvinyl alcohol (PVA).

The wet sheet 2, which is impregnated with chemicals containing distilled water or alcohol, is housed in the container body 11. The chemicals may contain sterilizer, fungicide, detergent, humectant and so on depending on applications. The wet sheet 2 impregnated with chemicals may be housed in the container body 11 without any packaging, but it is also possible to package the wet sheet 2 in a doughnut-shaped packaging bag of resin film and then house the wet sheet 2 in the container body 11 with the shafts 18, 28 inserted into a center hole of the packaging bag.

Thewet sheet container 10 may be set anywhere by supporting it through the support space 32. In the first embodiment shown in Figs. 1 to 3, for instance, the wet sheet container 10 is attached to the toilet paper holder 5. The holder 5 comprises a fixed plate 6 to be fixed to a wall surface of a bathroom by means of screws or the like, a support arm 7 extending from the fixed plate 6, and a cantilever rod 8 fixed to the support arm 7. Above the rod 8, there is provided a metal cover/cutter 9 which is pivoted at its end portion 9a on the fixed plate 6.

Hereinbelow, how to use the wet sheet container 10 will be described.

As shown in Fig. 1, after the first casing 11A and the second casing 11B are separated from each other, the sheet roll 1 is inserted into the opening 17 of the first casing 11A along the axial direction of the roll core. At this time, the shaft 18 is inserted into the center hole 4 of the sheet roll 1. Subsequently, the container body 11 is closed by a fit of the second casing 11B with the opening 17 of the first casing 11A, whereby the sheet roll 1 impregnated with liquid can be housed almost hermetically in the storage space 31.

The container body 11 can be supported by the holder 5 with the rod 8 of the holder 5 being inserted into the support space 32 of the container body 11. The container 10 thus supported is illustrated in Figs. 2 and 3.

When the wet sheet 2 is to be used, the lid 41 disposed in the front panel 13 is outwardly turned about the shaft 42 to open the dispensing opening 35. As the wet sheet 2 is pulled out through the dispensing opening 35, the sheet roll 1 rotates on the shafts 18, 28, enabling smooth pulling out of the wet sheet 2. Subsequently, the wet sheet 2 thus pulled out through the dispensing opening 35 is pulled upwardly or downwardly at an angle for tearing the wet sheet 2 along the perforation 3. The wet sheet 2 passing through the restriction hole 36 can be easily torn along the perforation 3 since the restriction hole 36 provides a resistance thereto as well as the wet sheet 2 is pressed against the upper or lower edge of the restriction hole 36.

In order to facilitate tearing of the wet sheet 2 as set forth above, the opening dimension of the restriction hole 36 in the X-direction is preferably smaller than the width of the wet sheet 2 in the X-direction, more preferably equal to or less than 2/3 the width of the wet sheet 2.

As thewet sheet 2 is being pulled out through the dispensing opening 35, the container body 11 is biased to rotate on the rod 8. At this time, the support portion 11E or 11F provided on the rear side of the container body 11 comes into contact with the fixed plate 6, preventing undesirable rotation of the container body 11.

Preferred dimensions of the wet sheet container 10 will be described hereinbelow. In order that the wet sheet container 10 can be certainly attached to the toilet paper holder 5 so as to prevent a fall, the dimension W of the container body 11 in the X-direction (see Fig. 2) is preferably in the range of 80 mm to 130 mm. In order that the rod 8 of the holder 5 can be inserted into the support space 32 formed in the container body 11, on the other hand, the minimum inner diameter D of the support space 32 is preferably in the range of 10 mm to 50 mm.

As shown in Fig. 3, the intermediate portion 11D and the support portions 11E, 11F on the rear side of the container body 11 are opposed to the fixed plate 6, wherein it is preferred that L1 is smaller than 80 mm and L2, L3 are equal to or larger than 80 mm so that the container body 11 can be certainly attached to the holder 5 and the support portions 11E, 11F can function to prevent rotation. It is more preferred that L1 is smaller than 60 mm and L2, L3 are equal to or larger than 60 mm.

Other embodiments of the present invention will be described hereinafter, wherein preferred dimensions are identical to those of the first embodiment. In the following embodiments, the detailed description of the portions having the same construction as those of the first embodiment will be omitted by designating them by the common reference numerals.

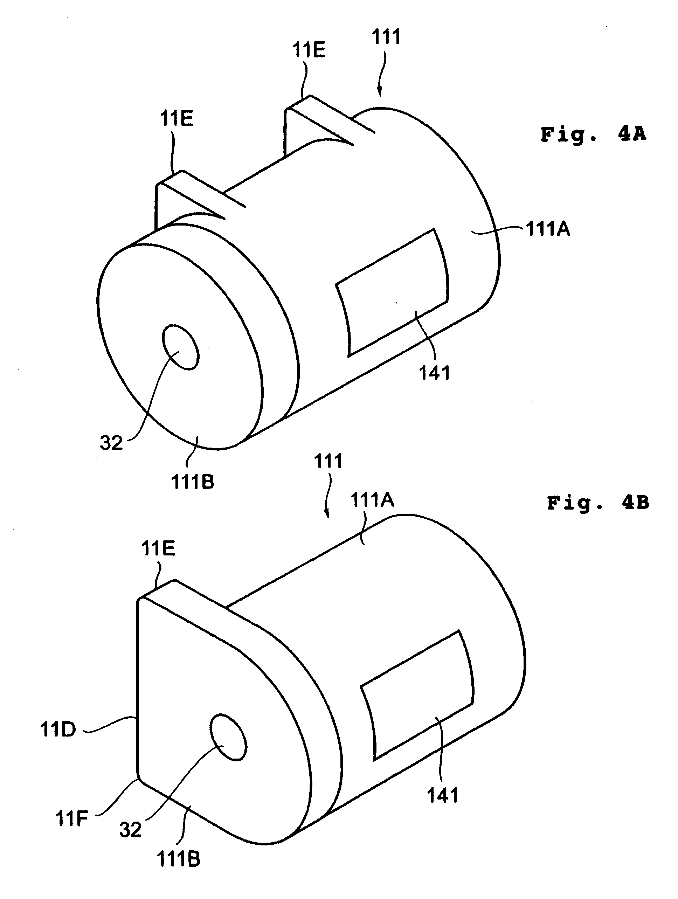

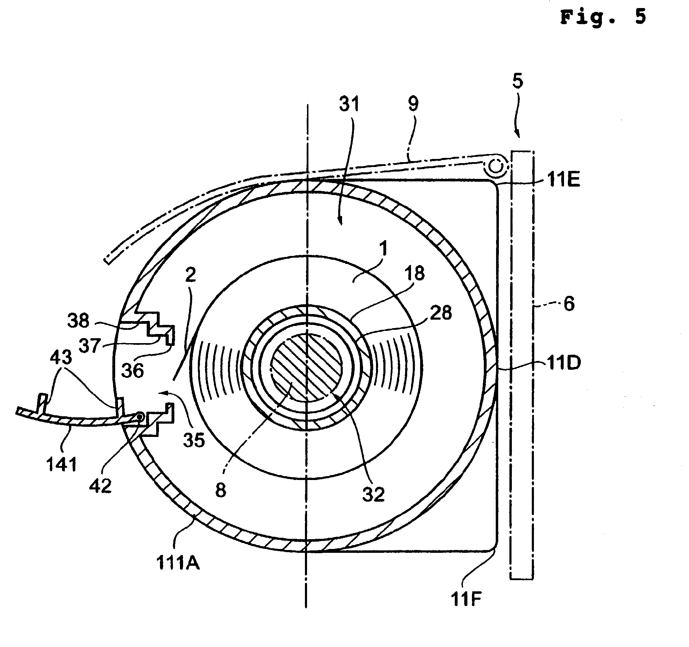

Figs. 4A and 4B are perspective views showing a container body 111 according to a second embodiment of the present invention. Fig. 5 is a sectional view showing a state where the container body 111 shown in Fig. 4A or 4B is attached to the toilet paper holder 5.

The container body 111 is separable into a first casing 111A and a second casing 111B, wherein the separation direction is along the axis Ox-Ox.

In Figs. 4A and 4B, both the first casing 111A and the second casing 111B are cylindrical except for a rib(s) that will be described hereinbelow. The shaft 18 is integrally formed inside the first casing 111A, while the shaft 28 is integrally formed inside the second casing 111B. In the front side of the first casing 111A, there is provided a lid 141 so that the dispensing opening 35 can be opened by turning the lid 141. Also in this embodiment, the sheet roll 1 can be housed almost hermetically in the storage space 31.

In Fig. 4A, a pair of ribs is formed on the rear side of the first casing 111A to provide the intermediate portion 11D and the support portions 11E, 11F for preventing rotation. In Fig. 4B, on the other hand, a single rib is integrally formed on the rear side of the second casing 111B to provide the intermediate portion 11D and the support portions 11E, 11F.

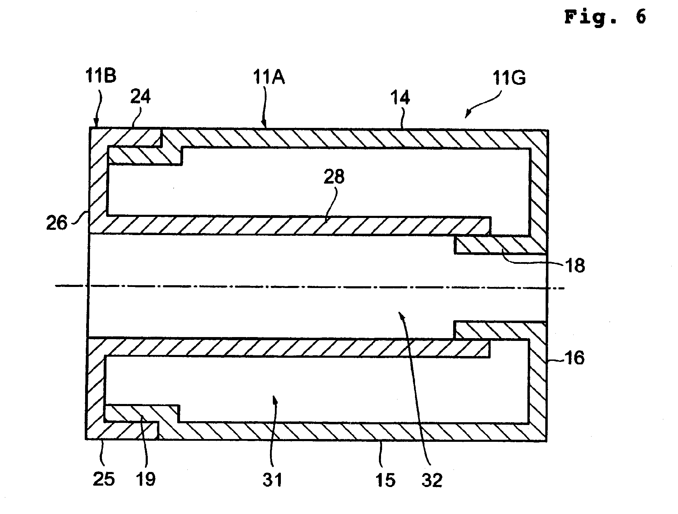

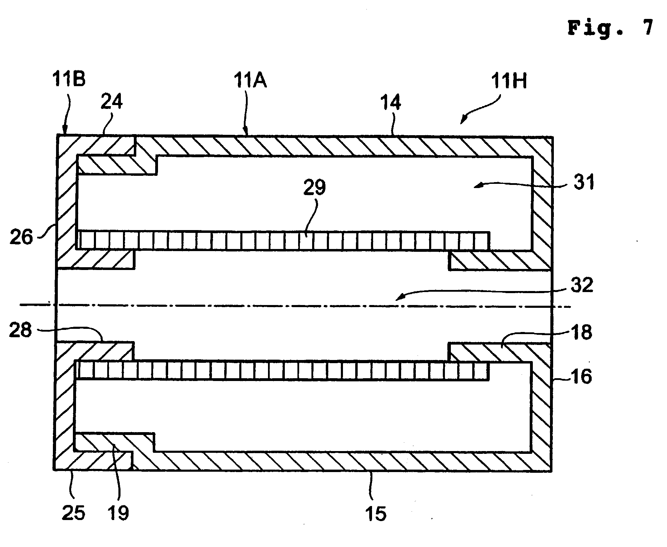

Fig. 6 is a sectional view similar to Fig. 2, showing a container body 11G according to a third embodiment of the present invention, and Fig. 7 is a sectional view similar to Fig. 2, showing a container body 11H according to a fourth embodiment of the present invention.

The container bodies 11G, 11H have the same appearance as the first embodiment, wherein the first casing 11A and the second casing 11B engage one another to provide the storage space 31 and the support space 32.

In the container body 11G of Fig. 6, the shaft 18 provided in the first casing 11A is relatively short and the shaft 28 provided in the second casing 11B is relatively long, wherein when the casings 11A, 11B engage one another, the support space 32 and the storage space 31 are separated from each other with the shafts 18, 28 engaging one another.

In the container body 11H of Fig. 7, both the shaft 18 provided in the first casing 11A and the shaft 28 provided in the second casing 11B are relatively short, wherein another hollow shaft 29 is fitted over the shafts 18, 28 to provide the support space 32 centrally of the hollow shaft 29.

Here, it is also possible to provide a cylindrical hollow core within the center hole 4 of the sheet roll 1 in advance. With such a hollow core surrounding the shaft 18, the shaft 28 or the hollow shaft 29, the sheet roll 1 housed in the storage space 31 can easily rotate to facilitate pulling out of the wet sheet 2.

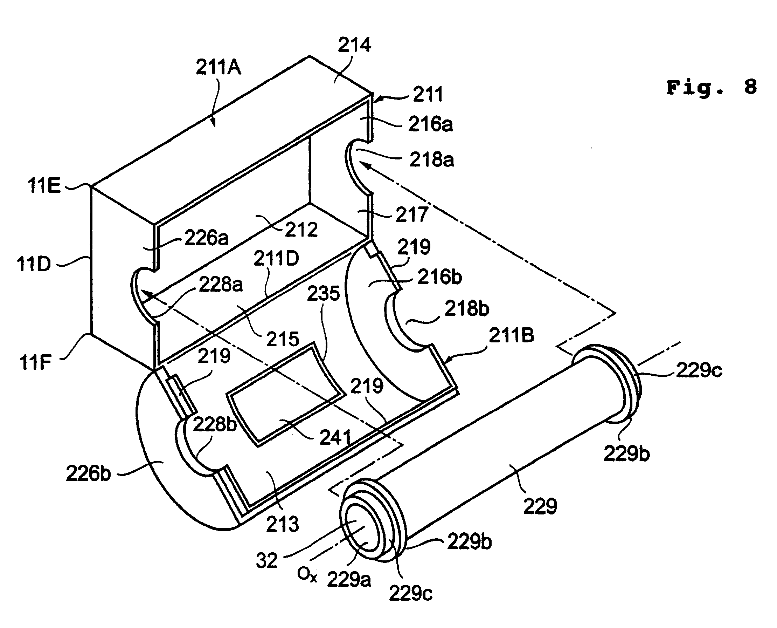

Fig. 8 is a perspective view showing a container body 211 according to a fifth embodiment of the present invention.

The container body 211 comprises a first casing 211A and a second casing 211B, which are pivotally connected to each other through a thin hinge 211D provided at a lower portion of the container body 211, so that the first casing 211A and the second casing 211B are separable in a direction perpendicular to the axis Ox-Ox. Here, it is also possible to provide a shaft to one of the first casing 211A and the second casing 211B and a bearing to the other, instead of providing the hinge, so that the casings are pivotally connected to each other through the shaft. Alternatively, the first casing 211A and the second casing 211B may be separated completely from each other for insertion of the sheet roll 1 without having the hinge 211D or the shaft and bearing.

The first casing 211A is box-shaped to have five panels: a rear panel 212, a top panel 214, a bottom panel 215 and end panels 216a, 226a, defining an opening 217 forward thereof. The second casing 211B has a hemicylindrical front panel 213 and end panels 216b, 226b. Along the edges of the front panel 213 and the end panels 216b, 226b, fitting ribs 219 are integrally formed so that when the second casing 211B is assembled to the first casing 211A, the fitting ribs 219 make a fit with the top panel 214 and the end panels 216a, 226a of the first casing 211A to keep airtightness of the container body 211.

The end panels 216a, 226a of the first casing 211A are formed with semicircular recesses 218a, 228a, respectively; the end panels 216b, 226b of the second casing 211B are formed with semicircular recesses 218b, 228b, respectively, so that when the second casing 211B is assembled to the first casing 211A, circular openings are formed in the end panels of the container body 211.

Inside the container body 211, there is disposed a shaft 229 separate from the first casing 211A and the second casing 211B. The shaft 229 defines therein a center hole 229a and ring-shaped ribs 229b, 229b are integrally formed adjacent opposite ends 229c, 229c to extend around the periphery of the shaft 229. With the opposite ends 229c, 229c of the shaft 229 being fitted in the recesses 218a, 218b and the recesses 228a, 228b so that the ribs 229b, 229b are in close contact with the inner surfaces of the end panels 216a, 216b and the inner surfaces of the end panels 226a, 226b, the container body 211 can be kept almost airtight.

Thus, when the first casing 211A, the second casing 211B and the shaft 229 are in their assembled condition, the almost airtight storage space 31 is formed to surround the shaft 229 and the center hole 229a of the shaft 229 functions as the support space 32 communicating with the outside of the container.

When the container body 211 is to be used, the first casing 211A and the second casing 211B are separated in a direction perpendicular to the axis Ox-Ox. Subsequently, the shaft 229 is inserted into the center hole 4 of the sheet roll 1, the sheet roll 1 is put in the first casing 211A together with the shaft 229, and then the second casing 211B is assembled to the first casing 211A. Finally, the container is set such that the rod 8 of the toilet paper holder 5 is inserted into the center hole 229a of the shaft 229 which functions as the support space 32.

As one way to take the wet sheet 2 out of the container body 211, the wet sheet 2 may be unwound from the sheet roll 1 exposed externally through the opening 217 which is opened by separating the second casing 211B from the first casing 211A as shown in Fig. 8. If thus used, it is not necessary to provide a dispensing opening in the second casing 211B.

However, in the embodiment shown in Fig. 8, the front panel 213 of the second casing 211B is formed with a dispensing opening 235, which is similar in structure to the dispensing opening 35 shown in Fig. 3, and a lid 241, which is similar in structure to the lid 41 shown in Fig. 3, is provided to be openable and closable. In this case, the wet sheet 2 can be pulled out through the dispensing opening 235 after the lid 241 is turned forward.

In the embodiment shown in Fig. 8, as set forth above, the sheet roll 1 can be put in the container body 211 together with the shaft 229 after the shaft 229 is inserted into the center hole 4 of the sheet roll 1. At this time, the sheet roll 1 impregnated with liquid can be housed in the container body 211, without directly touching the sheet roll 1, such that the opposite ends 229c of the shaft 229 are held with fingers or the shaft 229 is supported with a finger inserted into the center hole 229a, whereby the sheet roll 1 can be kept clean.

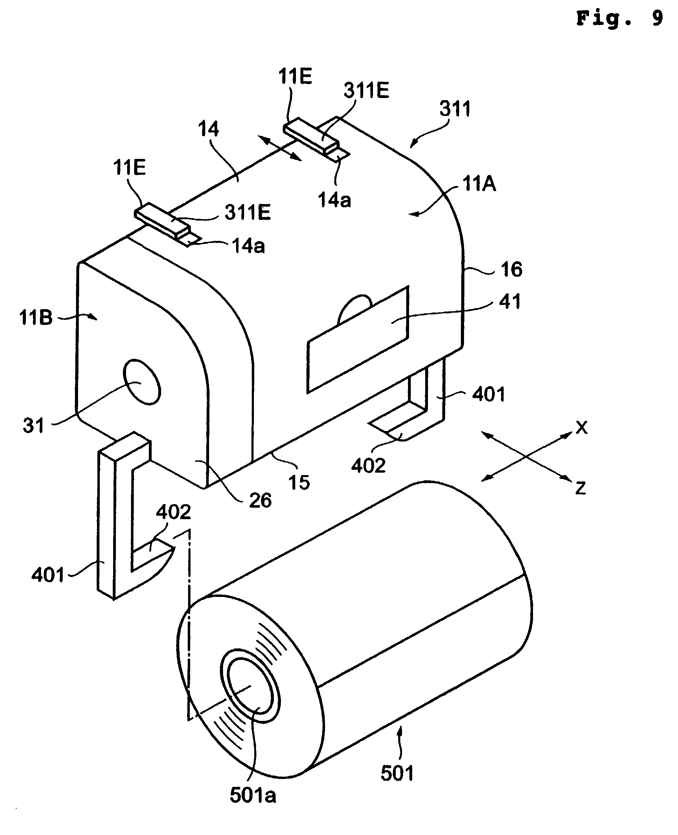

Fig. 9 shows a sixth embodiment of the present invention.

The embodiment shown in Fig. 9 provides a container body 311 whose basic structure is similar to that of the container body 11 shown in Figs. 1 to 3, wherein the first casing 11A and the second casing 11B are separable along the axis Ox-Ox. On the top panel 14 of the first casing 11A of the container body 311, support members 311E, 311E are disposed so as to be slidably movable in a direction perpendicular to the axis Ox-Ox. More particularly, slide grooves 14a, 14a are formed in the top panel 14 to extend in the Z-direction and the support members 311E, 311E are slidably fitted in the slide grooves 14a, 14a.

Similar support members (not shown) are disposed on the bottom panel 15 of the first casing 11A so as to be slidably movable in the Z-direction. As shown in Fig. 9, the leading ends of the support members 311E, 311E function as the support portion 11E. Likewise, the leading ends of the support members disposed on the bottom panel 15 function as the support portion 11F.

When the container body 311 is attached to the rod 8 of the toilet paper holder 5, the support members 311E, 311E and the support members disposed on the bottom panel 15 may be slid so that their leading ends abut on the fixed plate 6 of the holder 5 or a wall surface on which the holder 5 is installed, whereby rotation of the container body 311 on the rod 8 can be completely prevented.

It is, of course, possible to provide such support members also in the first to fifth embodiments.

In the embodiment of Fig. 9, furthermore, downwardly extending support arms 401, 401 are formed integral with or attached to the end panel 16 of the first casing 11A and the end panel 26 of the second casing 11B. Hooks 402, 402 are integrally formed on the lower ends of the support arms 401, 401. As shown in Fig. 9, a toilet paper 501 wound in the form of a roll may be supported with the hooks 402, 402 inserted into its core 501a for the use of the wet sheet 2 in combination with the toilet paper 501.

It is, of course, possible to provide the support arms 401 and the hooks 402 also in the first to fifth embodiments.

According to the present invention, as has been described hereinabove, the container body housing the wet sheet can be easily attached to the rod of the toilet paper holder or other arms. Since the support space formed in the container body, which communicates with the outside of the container body, is separated from the storage space, the container body can be kept airtight, preventing the wet sheet from drying.

Although the present invention has been illustrated and described with respect to exemplary embodiments thereof, it should be understood by those skilled in the art that the foregoing and various other changes, omission and additions may be made therein and thereto, without departing from the spirit and scope of the present invention. Therefore, the present invention should not be understood as limited to the specific embodiments set out above but to include all possible embodiments which can be embodied within a scope encompassed and equivalent thereof with respect to the feature set out in the appended claims.

## Claims

1. A wet sheet container for housing a sheet roll that is a wet sheet wound in the form of a roll, comprising:
a container body formed with a storage space into which the sheet roll is to be inserted, the container body being separable into two or more parts so that separation of the container body allows insertion of the sheet roll into the storage space; and
a shaft located inside the container body so as to be inserted into a center hole of the sheet roll, the shaft defining a support space which extends along an axial direction thereof and communicates with the outside of the container body.

2. A wet sheet container according to claim 1, wherein the container body is separable in the axial direction of the shaft so that separation of the container body allows insertion of the sheet roll into the storage space along an axial direction of the sheet roll, and the container body is formed with a dispensing opening for pulling out the wet sheet.

3. A wet sheet container according to claim 2, wherein a lid is provided to close the dispensing opening.

4. A wet sheet container according to claim 1, wherein the container body is separable in a direction perpendicular to the axial direction of the shaft so that separation of the container body allows insertion of the sheet roll into the storage space together with the shaft inserted into the center hole of the sheet roll.

5. A wet sheet container according to claim 4, wherein the container body is formed with a dispensing opening for pulling out the wet sheet.

6. A wet sheet container according to claim 5, wherein a lid is provided to close the dispensing opening.

7. A wet sheet container according to claim 1, wherein the support space formed inside the shaft allows insertion of a rod of a toilet paper holder, and the container body has a rear side formed with an intermediate portion and support portions at opposite sides of the intermediate portion in a rotational direction about an axis of the shaft, the support portions being located further away from the axis of the shaft than is the intermediate portion, functioning to prevent rotation of the container body.

8. A wet sheet container according to claim 1, wherein a paper support member for supporting a core of a toilet paper is provided outside the container body.
